(19) Europäisches Patentamt
European Patent Office
Office européen des brevets

(11) Publication number: 0 150 787 B1

(12) EUROPEAN PATENT SPECIFICATION

(45) Date of publication of patent specification: 27.04.88

(51) Int. Cl.$^4$: C 07 D 333/38, A 61 K 31/38

(21) Application number: 85100563.7

(22) Date of filing: 20.01.85

(54) **Compound having antibronchopneumopathic activity, process for its preparation and pharmaceutical compositions containing it.**

(30) Priority: 02.02.84 IT 1937484

(43) Date of publication of application: 07.08.85 Bulletin 85/32

(45) Publication of the grant of the patent: 27.04.88 Bulletin 88/17

(84) Designated Contracting States: AT BE CH DE FR GB IT LI LU NL SE

(56) References cited:
US-A-3 536 713

CHEMICAL ABSTRACTS, vol. 85, no. 19, 8th November 1976, page 497, no. 142791n, Columbus, Ohio, US; & ES - A 418 986 (LABORATORIOS ROGER S.A.) 01-03-1976

The file contains technical information submitted after the application was filed and not included in this specification

(73) Proprietor: PULITZER ITALIANA S.p.A.
Via Tiburtina, 1004
I-00156 Rome (IT)

(72) Inventor: Quadro, Giuseppe
Via Pisacane, 34/A
I-20129 Milan (IT)

(74) Representative: Bianchetti, Giuseppe
Studio Consulenza Brevettuale Via Rossini, 8
I-20122 Milan (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 150 787 B1

**Description**

The present invention relates to 4-[[(2-(2-thenoyl)-amino-3,5-dibromophenyl)-methyl]amino]cyclohexanol of formula I

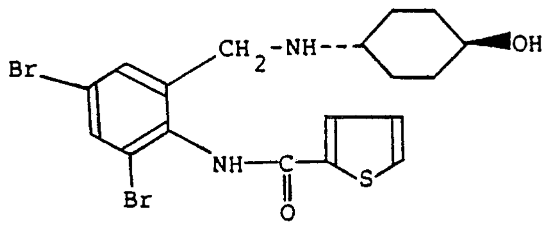

(I)

to a process for preparing it and to pharmaceutical compositions containing it as the active principle.

The invention relates also to pharmaceutically acceptable acid addition salts of compound I.

The hydrochloric salt of compound I, which will be hereinafter called PU 163, for sake of shortness, shows valuable pharmacological and pharmaco-kynetic properties and it is thus effective for the treatment both of acute and chronic respiratory diseases in humans, especially in case of qualitative or quantitative impairments in the mucous secretion, such as in bronchitis, bronchopneumonitis, bronchial asthma, pulmonary emphysema, tracheobronchitis, and the like.

The compound I proved to have better effectiveness and pharmacokynetis behaviour in comparison with known mucolytic drugs, namely Ambroxol.

Compound I may be prepared by reacting 4-[[(2-(2-amino-3,5-dibromophenyl)-methyl]amino]cyclohexanol (Ambroxol), a well known compound (*vide* U.S. Patent No. 3,536,713) currently used in therapy, with 2-thenoyl chloride in inert solvents, in the presence of acid-binding agents.

The nature of the solvent is not particularly critical, provided that it has no adverse effect upon the reaction; suitable solvents include benzene, toluene and pyridine.

Alkali carbonates or bicarbonates, tertiary amines etc. may be used as acid-binding agents. The reaction temperature is not critical, and it may range from +10°C and the reflux temperature of the solvent.

The acid addition salts may be obtained by conventioal salification methods, for instance the hydrochloric salt may be added by bubbling gaseous hydrochloric acid in an ethanol solution of compound I.

The following example further illustrates the process according to the invention.

Example

To a solution of Ambroxol in the free form (39 g; 0.1 mole) in toluene (350 ml) triethylamine (14 ml; 0.1 mole) was added, then 2-thenoyl chloride (14.6 ml; 0.1 mole) was added dropwise at room temperature. The reaction mixture was left to stand overnight, then the toluene solution was washed with water and the solvent was evaporated off.

The resulting residue (45 g) was dissolved in ethanol (250 ml) and gaseous hydrochloric acid was added to acid pH.

From the solution the hydrochloric salt precipitated, which was filtered and washed with ethanol, yielding 43 g of the desired compound; m.p. = 282—286°C.

Elemental analysis (for $C_{18}H_{20}Br_2N_2O_2S.HCl$, PM = 524.71)

| | C | H | N | S |
|---|---|---|---|---|
| Calc. % | 41.20 | 3.84 | 5.34 | 6.11 |
| Found % | 40.97 | 3.91 | 5.27 | 6.10 |

The NMR data agree with the structure of the compound.

NMR (δ): 1.1—2 (8H, m); 2.8—3.6 (3H, m); 3.8—4.2 (1H, m); 4.3—4.7 (1H, m); 7.2 (1H, t); 7.8—8.1 (3H, m); 8.3 (1H, d); 9.6 (2H, m); 10.3—10.7 (1H, m).

The toxico-pharmacological characteristics of compound I (PU 163) are documented by the following tests.

Ambroxol, active principle of several pharmaceutical specialities, which is 4-[[(2-amino-3,5-dibromophenyl)-methyl]amino]cyclohexanol (Merk Index, tenth edition, 383), was used as reference drug.

Acute toxicity

The acute toxicity of PU 163 has been determined in the mouse and in the rat by the oral route according to the method of Litchfield and Wilcoxon (J. Pharm. Exp. Therap. 1949, 96, 99).

The results are reported in the following Table 1.

TABLE 1

Acute toxicity

| Species | Administration route | $LD_{50}$ — mg/kg Ambroxol | PU 163 |
|---|---|---|---|
| mouse | os | >2,500 | >2,500 |
| rat | os | >5,000 | >5,000 |

Pharmacology

*Action on the ciliary motility in the dog's trachea*

The mucolytic activity of many products is due in that they induce an increase of motility in the trachea cilitate cells.

The method hereinafter described allows to assess "in vitro" if a compound increases, or not, the motility of said cells. The method is based on the measurement of the transit speed of a charcoal bolus in a section of trachea.

The method used is that described by J. E. Lightowler and N. H. Lightowler (Arch. Int. Pharmacodyn. *189,* 53—58, 1971) and uses an apparatus similar to that described by T. Dalhamn (Acta Physiol. Scand. Suppl. 123, 1956).

PU 163 has been used in comparison with Ambroxol, at the same concentration of $10^{-4}$ g/ml and the results deriving from the test are reported in Table 2, from which it can be shown that PU 163 exhibits an action on the ciliary motility to a higher extent than that of Ambroxol, but of the same order of significance.

TABLE 2

Mucolytic activity — Ciliary motility on the dog's trachea

| Treatment | Concentration g/ml | No. Animals | Speed of Charcoal Particles | | | | | | |
|---|---|---|---|---|---|---|---|---|---|
| | | | | 5 Minutes | | 10 Minutes | | 20 Minutes | |
| | | | Basal mm/min. $\bar{x} \pm$ ES | mm/min. $\bar{x} \pm$ ES | % Δ vs. Bas. $\bar{x} \pm$ ES | mm/min. $\bar{x} \pm$ ES | % Δ vs. Bas. $\bar{x} \pm$ ES | mm/min. $\bar{x} \pm$ ES | %Δ vs. Bas. $\bar{x} \pm$ ES |
| Controls | — | 4 | 5.2 ±1.03 | 6.3 ±1.36 | 21.1 ±3.38 | 6.02 ±1.07 | 19.2 ±6.25 | 5.8 ±1.04 | 11.5 ±3.82 |
| Ambroxol | $10^{-4}$ | 4 | 6.4 ±1.30 | 8.2 ±2.12 | 28.12 ±3.54 | 9.5 ±1.62 | 57.6* ±14.60 | 10.2 ±2.31 | 59.37** ±8.12 |
| PU 163 | $10^{-4}$ | 4 | 7.00 ±1.40 | 8.7 ±2.82 | 24.28 ±7.80 | 10.2 ±2.02 | 45.7* ±8.40 | 11.5 ±2.38 | 64.28** ±9.12 |

Student's "t" test versus buffer solution * $p < 0.05$
** $p < 0.01$
*** $p < 0.001$

*Bronchosecretogogue activity*

The bronchosecretogogue activity of PU 163 and, as a comparison, of Ambroxol has been determined in the rat using the method described by Mawatari ("Experimental Studies on the Expectorant Action of Several Drugs", Kagoshima Daigaku Igaku Zasshi, *27,* 561, 1976).

The compounds under exam have been administered by the oral route at equiponderal doses of 50 mg 30 minutes after the subcutaneous administration of sodium fluorescein.

After the animal's sacrifice, the evaluation of the amount of sodium fluorescein present at the bronchial level has been carried out. The results obtained, reported in the Table 3, are expressed as percent increase of the bronchial elimination of sodium fluorescein of the groups treated with Ambroxol and PU 163 in comparison with a control group.

As it can be seen, PU 163 showed a high bronchosecretogogue activity, in slightly higher extent than that of Ambroxol in the used experimental conditions.

TABLE 3

Bronchosecretogogue activity in the rat (Mawatari test)

| Treatment | Dose mg/kg i.p. | No. of Animals | Animals Weight/ grams | Sodium Fluorescein | |
|---|---|---|---|---|---|
| | | | | μg/ml | % vs. control |
| Controls | — | 10 | 96.0 ±5.56 | 0.28 ±0.02 | — |
| Ambroxol | 50 | 10 | 98.0 ±7.41 | 0.37* ±0.05 | 32.1 |
| PU 163 | 50 | 10 | 102.0 ±2.91 | 0.39* ±0.06 | 39.3 |

Student's "t" test *$p < 0.05$

*Activity on the alveolar surfactant*

This test allows to verify if a compound can modify the surface of lung alevoli, as there are pathological conditions in which a superficial firm is produced in the alveoli which, as time goes on, can produce foam in the bronchi, making the respiration difficult.

In order to assess this kind of activity, the method described by Curty P. C. (Arzneim. Forsch. Drug. Res., *24,* 847, 1974) is used. According to said method the volume and the pressure after insufflation in the rat's lung, are measured.

PU 163 and Ambroxol as reference drug have been administered at equiponderant doses of 50 mg/kg in the rat by oral route, three hours before the test. After sacrifice, and after the trachea incannulation, the respiratory apparatus has been extracted from the thoracic cavity and the static curves of volume-pulmonary pressure.

From the test results, as it is shown in the following Table 4, it is found that both the compounds under exam decrease the pulmonary pressure induced by air insufflation in marked way in comparison with the control animals. At the same time, however, the recovery of the basal pressure in the insufflation phase is shorter for PU 163 both in comparison with the controls and with Ambroxol and in the de-insufflation phase the recovery of PU 163 is longer both in comparison with the controls and in comparison with Ambroxol. Therefore, in the used experimental conditions, PU 163 seems to be endowed with a higher activity on the test of the alveolar surfactant.

TABLE 4

Activity of the alveolar surfactant in the rat — Static insufflation and de-insufflation rates

| Treatment | Dose mg/kg p.o. | No. of animals | $\bar{x}$ Weight g | Internal pulmonary pressure (Pascal) | | | | | | | | | | | | |
|---|---|---|---|---|---|---|---|---|---|---|---|---|---|---|---|---|
| | | | | 0.5 | 1 | 1.5 | 2 | 2.5 | 3 | 3.5 | 3 | 2.5 | 2 | 1.5 | 1 | 0.5 $cm^3$ |
| Controls | — | 5 | 266.0 ±5.10 | 294.5 | 490.3 | 652.1 | 813.9 | 980.7 | 1049.3 | 1137.6 | 764.9 | 519.8 | 313.8 | 137.3 | 24.5 | |
| Ambroxol | 50 | 5 | 245.0 ±7.20 | 240.3 | 402.1 | 549.2 | 676.7 | 774.7 | 853.2 | 912.0 | 598.2 | 451.1 | 323.6 | 225.6 | 107.9 | 24.5 |
| PU 163 | 50 | 5 | 238.0 ±5.00 | 215.7 | 372.7 | 524.7 | 637.4 | 745.3 | 828.7 | 882.6 | 661.9 | 509.9 | 382.5 | 245.2 | 137.3 | 34.3 |

*Action on the pulmonary emphysema*

The ability of PU 163 of antagonizing the pulmonary emphysema induced in the guinea pig by intratracheal injection of 0.1 mg of porcine pancreatic elastase has been evaluated in comparison with Ambroxol, according to the method described by Stone P. J. et al. (Am. Rev. Resp. Dis. 124/1, 56—59; 1981). Both compounds have been administered by the i.p. route at the equiponderant dose of 50 mg/kg 1 hour after the elastase injection. From the results found, PU 163 turned out to be endowed with a high activity of reducing emphysema, in remarkably higher extent than that found for Ambroxol.

*Action on the chronic bronchitis*

The action of PU 163 in comparison with Ambroxol on the chronic bronchitis induced by inhalation of $SO_2$ in the rat has been evaluated according to the method described by Quevauvillier e coll. (Therapie, *XXII;* 185—193; 1967).

The compounds under exam have been administered by gastric tube at the dose of 50 mg/kg, suspended in carboxymethylcellulose. The efficacy of the treatment has been evaluated in function of the regression degree of the pulmonary lesions induced by $SO_2$ inhalation.

The results showed that both compounds exert a good activity on the pathological signs induced by the chronic bronchitis, but to a remarkably higher extent in the group of PU 163 treated animals.

In fact, according to the evaluation of the lesions degrees, PU 163 showed a percent regression of 62%, compared to 38% for the Ambroxol treated group at equiponderant doses.

*Action on the inhibition by elastae*

The inhibiting activity of PU 163 and of Ambroxol on elastase has been measured according to the method described by R. M. Senior (Plenum Press. New York; 249—254; 1977). According to the results, PU 163 showed a potent elastolytic inhibiting activity with an $IC_{50}$ corresponding to 11 mM/l. In this test, Ambroxol turned out to be practically inactive.

*Pharmacokinetics and distribution*

The pharmacokynetics behaviour and the distribution in the organs of PU 163, in comparison to Ambroxol, has been determined in the rat after administration by the oral route of equimolar doses, corresponding to 50 mg/kg of Ambroxol base.

The assays have been performed by HPLC and the results clearly showed some fundamental advantages of PU 163 in comparison with Ambroxol.

— PU 163 can be assayed in the plasma as such in the first detection times, to give them Ambroxol; the concentrations as Ambroxol in the PU 163 treated group can be detected at prolonged times in comparison with the Ambroxol treated group. This pattern of the plasma concentrations allows a more prolonged presence in the circulation of Ambroxol, so as to allow a reduction of the daily administrations' frequence.

— Form the exam of the concentrations in the organs it was surprisingly found how the structure of PU 163 allows to carry larger amounts of Ambroxol at the pulmonary level, target organ for the therapeutic applications envisaged for the drug's use.

The invention also provides pharmaceutical compositions containing, as the active ingredient, prefixed and therapeutically effective amounts of compound I or its pharmaceutically acceptable addition salts as antibronchopneumoniae agents, in admixture with a pharmaceutically acceptable carrier or diluent to be administered orally, rectally, parenterally or by inhalatory route.

Examples of such formulations are tablets, capsules, pills, powders, granules, lozenges, syrups, suppositories, phials, ampoules, aerosols, possible sustained release dosage forms, obtained for instance by microincapsulation, commonly used in pharmaceutical technique, together with suitably excipients, disintegrating agents, tabletting aids, flavouring agents, colouring matter and other diluents as required.

The preferred individual dosage unit will vary, depending upon the weight of the patient and the severity of the disease; generally it will range from 5 to 50 mg of compounds of the invention, in admixture with suitable carriers.

Such dosage units will be administered from 2 to 4 times a day.

**Claims for the Contracting States: BE CH DE FR GB IT LI LU NL SE**

1. 4-[[(2-(2-Thenoyl)amino-3,5-dibromophenyl)-methyl]amino]cyclohexanol of formula I

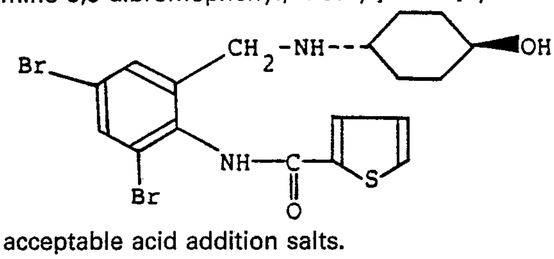

(I)

and its pharmaceutically acceptable acid addition salts.

2. 4-[[(2-(2-Thenoyl)amino-3,5-dibromophenyl)-methyl]amino]cyclohexanol hydrochloride.

3. A process for preparing compound I, which comprises reacting 4-[[(2-amino-3,5-dibromophenyl)-methyl]amino]cyclohexano with 2-thenoyl chloride, in the presence of aromatic hydrocarbons and in the presence of alkali carbonates or bicarbonates, or tertiary amines, acting as acid-binding agents.

4. A process according to claim 3, in which the reaction is effected at temperatures ranging from 10°C to the reflux temperature of the solvent.

5. Pharmacuetical compositions having antibronchopneumopathic activity, comprising as the active ingredient a compound of claims 1—2.

6. Pharmaceutical compositions according to claim 5, in form of capsules, tablets, suppositories, syrups, lozenges, powders, solutions.

**Claims for the Contracting State: AT**

1. A process for preparing compound I

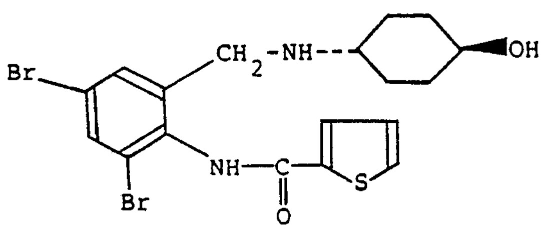

(I)

which comprises reacting 4-[[(2-amino-3,5-dibromophenyl)-methyl]amino]cyclohexanol with 2-thenoyl chloride, in the presence of aromatic hydrocarbons, and in the presence of alkali carbonates or bicarbonates, or tertiary amines, acting as acid-binding agents.

2. A process according to claim 1, in which the reaction is effected at temperatures ranging from 10°C to the reflux temperature of the solvent.

**Patentansprüche für die Vertragsstaaten: BE CH DE FR GB IT LI LU NL SE**

1. 4-[[(2-(2-Thenoyl)amino-3,5-dibromphenyl)-methyl]-amino]cyclohexanol der Formel I

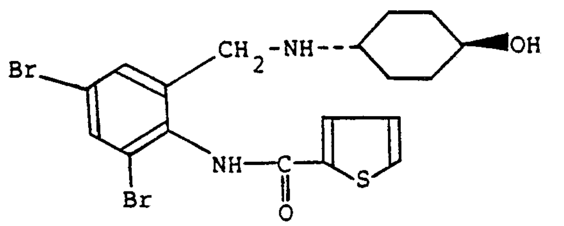

(I)

und dessen pharmazeutisch undebenklichen Säureadditionssalze.

2. 4-[[(2-(2-Thenoyl)amino-3,5-dibromphenyl)-methyl]-amino]cyclohexanol-hydrochlorid.

3. Verfahren zur Herstellung der Verbindung I, wobei 4-[[(2-Amino-3,5-dibromphenyl)-methyl]amino]-cyclohexanol mit 2-Thenoylchlorid in Gegenwart von aromatischen Kohlenwasserstoffen und in Gegenwart von Alkalicarbonaten oder -bicarbonaten oder tertiäaren Aminen, die als Säurebindungsmittel dienen, umgesetzt wird.

4. Verfahren nach Anspruchen 3, worin die Reaktion bei Temperaturen in Bereich von 10°C bis zur Rückflußtemperatur des Lösungsmittels durchgeführt wird.

5. Pharmazeutische Zusammensetzungen mit antibronchopneumopathischer Wirksamkeit, welche als den aktiven Bestandteil eine Verbindung nach den Ansprüchen 1 bis 2 enthalten.

6. Pharmazeutische Zusammensetzungen nach Anspruch 5 in Form von Kapseln, Tabletten, Suppositorien, Sirupen, Pastillen, Pulvern, Lösungen.

**Patentansprüche für den Vertragsstaat: AT**

1. Verfahren zur Herstellung der Verbindung I

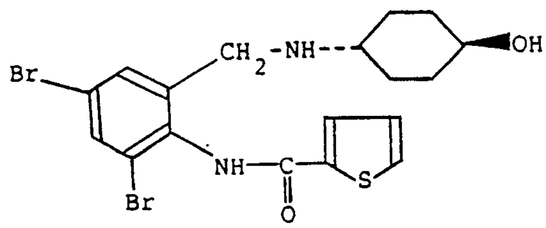

(I)

wobei 4-[[(2-Amino-3,5-dibromphenyl)-methyl]amino]-cyclohexanol mit 2-Thenoylchlorid in Gegenwart von aromatischen Kohlenwasserstoffen und in Gegenwart von Alkalicarbonaten oder -bicarbonaten oder tertiären Amine, die als Säurebindungsmittel dienen, ungesetzt wird.

2. Verfahren nach Anspruch 1, worin die Reaktion bei Temperaturen im Bereich von 10°C bis zur Rückflußtemperatur des Lösungsmittels durchgeführt wird.

**Revendications pour les Etats contractants: BE CH DE FR GB IT LI LU NL SE**

1. [[(2-(2-Thénoyl)amino-3,5-dibromophénil)-méthyl]amino]cyclohexanol de formule I:

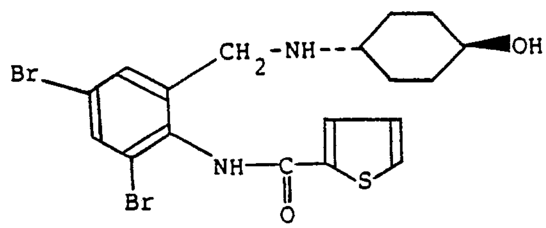

(I)

et ses sels d'addition acides pharmaceutiquement acceptables.

2. Hydrochlorure de 4-[[(2-(2-thénoyl)amino-3,5-dibromophényl)-méthyl]amino]cyclohexanol.

3. Procédé pour préparer le composé I, qui comprend une réaction du 4-[[(2-amino-3,5-dibromophényl)-méthyl]amino]cyclohexanol avec le chlorure de 2-thénoyle, en présence de hydrocarbures aromatiques et en présence de carbonates ou de bicarbonates alcalins, ou d'amines tertiaires, agissant comme agents de liaisons acides.

4. Procédé selon la revendication 3, dans lequel la réaction est effectuée à des températures variant de 10°C à la température de reflux du solvant.

5. Compositions pharmaceutiques ayant une activité antibronchopneumopathique, comprenant en tant qu'ingrédient actif un composé des revendications 1 ou 2.

6. Composition pharmaceutiques selon la revendication 5, en forme de capsules, comprimés, suppositoires, sirops, pastilles, poudres, solutions.

**Revendications pour l'Etat contractant: AT**

1. Procédé pour préparer le composé I:

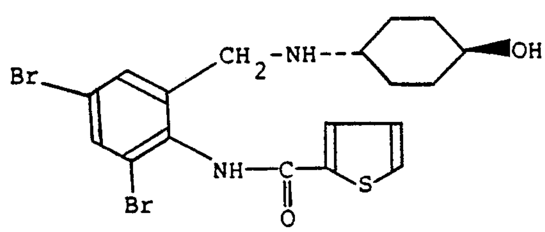

(I)

qui comprend une réaction du 4-[[(2-amino-3,5-dibromophényl)-méthyl]amino]cyclohexanol avec le chlorure de 2-thénoyle, en présence de hydrocarbures aromatiques et en présence de carbonates ou de bicarbonates alcalins, ou d'amines tertiaires, agissant comme agents de liaisons acides.

2. Procédé selon la revendication 1, dans lequel la réaction est effectuée à des températures variant de 10°C à la température de reflux du solvant.